Europäisches Patentamt

European Patent Office

Office européen des brevets

(11) Veröffentlichungsnummer: **0 165 457**
A2

# (12) EUROPÄISCHE PATENTANMELDUNG

(21) Anmeldenummer: 85105856.0

(22) Anmeldetag: 13.05.85

(51) Int. Cl.⁴: **A 61 K 7/00**
**A 61 K 47/00**

(30) Priorität: 21.05.84 DE 3418887

(43) Veröffentlichungstag der Anmeldung:
27.12.85 Patentblatt 85/52

(84) Benannte Vertragsstaaten:
AT BE CH DE FR GB IT LI LU NL SE

(71) Anmelder: Henkel Kommanditgesellschaft auf Aktien
Postfach 1100 Henkelstrasse 67
D-4000 Düsseldorf-Holthausen(DE)

(72) Erfinder: Ploog, Uwe, Dr.
Haydnweg 6
D-5657 Haan(DE)

(72) Erfinder: Scheuffgen, Ingeborg
Spulgasse 3
D-4040 Neuss(DE)

(54) Kosmetische Ölkomponente.

(57) n-Decylerucat ist allein oder mit bis zu 20 Gew. -% Estern gesättigter und ungesättigter Fettsäuren mit 16 − 20 C-Atomen mit gesättigten Fettalkoholen mit 6 − 12 C-Atomen als gut spreitende und stabile Ölkomponente für kosmetische und pharmazeutische Zubereitungen geeignet. Cremes und Emulsionen enthalten bevorzugt 5 − 40 Gew.-% einer bei 20°C flüssigen Ölkomponente, die zu 50 − 100 Gew.-% aus n-Decylerucat besteht.

EP 0 165 457 A2

Henkelstraße 67

4ooo Düsseldorf, den 17.5.1984

**0165457**
**HENKEL KGaA**
ZR-FE/Patente
Dr.JG/Po

P a t e n t a n m e l d u n g

D 7014 EP

"Kosmetische Ölkomponente"

Gegenstand der Erfindung ist eine neue Ölkomponente für kosmetische und pharmazeutische Anwendungen.

Ölige Flüssigkeiten natürlicher oder synthetischer Herkunft bilden die Grundlage zahlreicher kosmetischer und pharmazeutischer Zubereitungen wie Hautöle, Cremes, Salben, flüssige Emulsionen und Stiftpräparate. Sie erfüllen in diesen Mitteln verschiedene Aufgaben: Sie sollen der Haut nach der Anwendung Glätte und Geschmeidigkeit verleihen, ihr nach dem Waschen Fettstoffe zuführen, ihr Austrocknen verhindern und als Lösungsmittel und Träger der verschiedenen kosmetischen und dermatologischen Wirkstoffe und/oder Pigmente dienen.

Als Ölkomponenten werden sowohl natürliche, pflanzliche und tierische Öle wie z.B. Olivenöl, Erdnußöl, Mandelöl, Jojobaöl, Rizinusöl, Nerzöl, Spermöl als auch mineralische Öle, z.B. Paraffinöle eingesetzt. Brauchbar sind auch Ester natürlicher Fettsäuren und einwertiger oder mehrwertiger Alkohole sowie modifizierte Naturstoffe wie hydriertes Squalen oder hydrierte Terpene.

Bekannte Ölkomponenten sind auch ungesättigte Fettstoffe, z.B. der Oleylalkohol, die Ester des Oleylalkohols wie z.B.   Oleyloleat und andere Ester der Ölsäure wie z.B. Butyloleat und Decyloleat. Solche ungesättigten Fettkörper weisen ein hohes Spreitvermögen auf der Haut auf und zeichnen sich durch eine gute Verteilbarkeit, ein gutes Einziehvermögen in die Haut und eine gute Verträglichkeit aus. Diese Eigenschaften mögen eine Folge der strukturellen Verwandtschaft zu den biologischen Hautfetten sein. Nachteilig ist jedoch die Neigung der ungesättigten Fettderivate zur Autoxidation und Ranzidität. Nachteilig ist auch die Schwierigkeit, solche ungesättigten Öle in stabile Emulsionen zu überführen.

Es wurde nun gefunden, daß die genannten Nachteile der Oleylalkohol- und Ölsäurederivate bei einer Ölkomponente für kosmetische und pharmazeutische Zubereitungen kaum bzw. in erheblich geringerem Umfange auftreten, wenn diese ganz oder überwiegend aus n-Decylerucat besteht.

n-Decylerucat der Ester aus n-Decanol-1 und der 13 c-Docosensäure (Erucasäure) weist aber die vorgenannte Vorteile - das hohe Spreitvermögen, die gute Verteilbarkeit, das gute Einziehvermögen und die gute Verträglichkeit auf der Haut - in besonders hohem Maße auf und ist daher als flüssige, kosmetische und/oder pharmazeutische Ölkomponente bevorzugt geeignet.

Die vorzüglichen anwendungstechnischen Eigenschaften werden auch mit "technischem" n-Decylerucat, d.h. mit

einem aus einem technischen n-Decanol-1 mit den üblichen geringfügigen Beimengungen an n-Hexanol-1, n-Octanol-1 und n-Dodecanol-1 und einer technischen Erucasäure mit geringfügigen Beimengungen an z.B. Gadoleinsäure, Ölsäure, Linolsäure, Stearinsäure, Palmitinsäure und Palmitoleinsäure zugänglichen Ester erhalten.
Bevorzugt sollte die erfindungsgemäße Ölkomponente
wenigstens 80 Gew.-% n-Decylerucat neben kleineren
Mengen von 1 - 20 Gew.-% an Estern von gesättigten
und ungesättigten Fettsäuren mit 16 - 20 C-Atomen mit
gesättigten Fettalkoholen mit 6 - 12 C-Atomen enthalten.

n-Decylerucat läßt sich durch einfache Veresterung von
n-Decanol-1 mit Erucasäure (13 c-Docosensäure) herstellen. Die Veresterung wird in Gegenwart eines Veresterungskatalysators, z.B. von Alkalimetallhydroxid,
-carbonat, -acetat, eines Erdalkalimetalloxids, -hydroxids, -carbonats, einer organischen Titanverbindung, z.B. Tetraisopropyltitanat oder Zinn, z.B. in
Form von Zinnpulver durchgeführt. Geeignet sind auch
saure Veresterungskatalysatoren wie z.B. Schwefelsäure,
organische Sulfonsäuren oder saure Jonenaustauscher.
Zur Veresterung werden die Komponenten in Gegenwart
des Katalysators auf eine Temperatur von ca. 150 -
250°C erhitzt, wobei das Reaktionswasser sich abspaltet und bevorzugt unter vermindertem Druck aus dem
Reaktionsgemisch entfernt wird.

Anstelle von n-Decanol-1 kann auch ein technischer
Decylalkohol mit Anteilen an n-Hexanol-1, n-Octanol-1
und n-Dodecanol-1 in Mengen von bis zu insgesamt 10
Gew.-% dieser Alkohole eingesetzt werden. Anstelle

...

von reiner Erucasäure kann auch eine technische Erucasäure verwendet werden, wie sie aus dem (erucasäurereichen) Rüböl bzw. Rapsöl durch Verseifung und Fraktionierung gewonnen werden kann. Eine solche technische
Erucasäure enthält ca. 80 - 95 Gew.-% Erucasäure neben
geringeren Mengen an Ölsäure, Linolsäure, Linolensäure,
Gadoleinsäure, Stearinsäure und Palmitinsäure.

n-Decylerucat bzw. kosmetische und pharmazeutische Ölkomponenten, die überwiegend aus n-Decylerucat bestehen,
weisen neben den bereits beschriebenen Vorteilen ein
hohes Lösevermögen für öllösliche kosmetische und pharmazeutische Wirkstoffe auf. Sie lassen sich mit den bekannten Emulgatoren und Emulgator-Kombinationen problemlos in flüssige Emulsionen und in Cremes und Salben
- sowohl vom Typ Öl-in-Wasser (O/W) als auch vom Typ
Wasser-in-Öl (W/O) überführen. Solche Emulsionen mit
n-Decylerucat oder einer überwiegend aus n-Decylerucat
bestehenden flüssigen Ölkomponente zeichnen sich durch
hohe Stabilität, auch gegenüber Temperaturbelastung,
und durch gute Verteilbarkeit auf der Haut aus. Gegen
Autoxidation und Ranzidität sind solche Zubereitungen
weniger anfällig als solche mit bekannten, ungesättigten Ölkomponenten. Dieser Befund ist im Hinblick auf
die chemische Verwandtschaft mit Estern der Ölsäure
besonders überraschend.

Erfindungsgemäße kosmetische Cremes und Emulsionen enthalten bevorzugt 5 - 40 Gew.-% einer bei + 20°C flüssigen Ölkomponente, die zu 50 - 100 Gew.-% aus n-Decylerucat besteht. Neben n-Decylerucat können einerseits die im technischen n-Decylerucat vorhandenen Ester ungesättigter und gesättigter Fettsäuren mit
16 - 20 C-Atomen mit gesättigten Fettalkoholen mit
6 - 12 C-Atomen enthalten sein. Andererseits können
weitere flüssige Ölkomponenten, bevorzugt in geringeren
Anteilen, z.B. gesättigte $C_8$ - $C_{10}$-Fettsäuretriglyceride,
flüssige Pflanzenöle, Polyol-Fettsäureester (z.B. Fettsäureester von oxethyliertem Glycerin), Guerbet-Alkohole wie z.B. 2-Octyl-dodecanol, gesättigte Fettsäureester wie z.B. Isopropylmyristat, Isopropylstearat,
Hexyllaurat, Paraffinöle oder Silikonöle enthalten
sein.

Neben den flüssigen Ölkomponenten können zur Verbesserung
der Konsistenz und Stabilität noch wachsartige Fettstoffe und konsistenzgebende Komponenten in den Emulsionen und Cremes enthalten sein, z.B. Bienenwachs,
Wollwachs, Palmitinsäure-/Stearinsäure-partialglyceride,
Walrat oder Walratsubstitute, Vaseline und/oder gesättigte Fettalkohole mit 16 - 22 C-Atomen (z.B. Cetyl-/
Stearylalkohol).

Als Emulgatoren zur Herstellung von Emulsionen und Cremes
des Öl-in-Wasser-(O/W)-Typs kommen z.B. oxethylierte
Fettalkohole (z.B. Cetyl-/Stearylalkohol mit 10 - 30
Mol EO),oxethylierte Fettsäurepartialglyceride, oxethylierte Sorbitanfettsäureester, Seifen oder Fettalkoholsulfate (z.B. Cetyl-/Stearylsulfat) in Frage. Cremes
vom Typ Wasser-in-Öl werden bevorzugt mit lipophilen
Emulgatoren von niedrigem HLB-Wert hergestellt. Solche
geeignete W/O-Emulgatoren sind z.B. Fettsäurepartialglyceride (z.B. Palmitin-/Stearinsäure - Mono-/Digly-
cerid), Sorbitanfettsäureester (z.B. Sorbitansesquioleat), Trialkylphosphate z.B. Trioleylphosphat, Woll-

...

wachs und Mischester aus Pentaerythrit, Fettsäure, Zitronensäure und Fettalkohol wie sie z.B. aus DE-PS 1.165.574 bekannt sind.

Die Emulgatoren werden in einer Menge von 10 - 60 Gew.-%, bezogen auf die Fettphase der Emulsion oder Creme (bestehend aus Ölkomponente, konsistenzgebenden Faktoren und öllöslichen Emulgatoren und öllöslichen kosmetischen oder pharmazeutischen Wirkstoffen und Duftstoffen) eingesetzt.

Die wäßrige Phase der erfindungsgemäßen Emulsionen und Cremes enthält neben Wasser in geringen Mengen Glycerin, 1.2-Propylenglykol, ggf. wasserlösliche Polymere, wie z.B. Carboxymethylcellulose, Hydroxyethylcellulose, Carboxyvinylpolymere (z.B. Carbopol$^{(R)}$) und ggf. Triethanolamin, wasserlösliche Konservierungsmittel, wasserlösliche kosmetische Wirkstoffe (Vitamine, Pflanzenextrakte) und ggf. wasserlösliche Emulgatoren. Die erfindungsgemäßen Cremes und Emulsionen setzen sich zusammen aus 15 - 50 Gew.-% Fettphase und 50 - 85 Gew.-% wäßriger Phase. Ihre Herstellung erfolgt in üblicher Weise dadurch, daß die Komponenten der Fettphase miteinander nach Erwärmen über den gemeinsamen Schmelzpunkt im flüssigen Zustand (üblicherweise bei ca. 60 - 80°C) vermischt werden und die Komponenten der wäßrigen Phase nach Erwärmung auf 60 - 80°C unter intensivem Rühren und ggf. unter Verwendung bekannter Emulgiervorrichtungen in die erwärmte Fettphase nach und nach eingearbeitet werden. Leicht flüchtige Komponenten, wie z.B. ätherische Öle und Duftstoffe werden bevorzugt kurz vor oder während der Abkühlung in die Emulsionen eingearbeitet.

...

- 7 -

Ein weiteres bevorzugtes Anwendungsgebiet für die erfindungsgemäße neue Ölkomponente ist das der wasserfreien, flüssigen, kosmetischen und pharmazeutischen Zubereitungen. Solche Präparate sind z.B. Hautöle, Sonnenöle, Massageöle, Babyöle, Abschmink- und Reinigungsöle, Nagelpflegeöle, Haaröle und ggf. Badeöle. Solche wasserfreien flüssigen Zubereitungen enthalten bevorzugt 20 - 60 Gew.-% n-Decylerucat, 20 - 60 Gew.-% Paraffinöl und gegebenenfalls weitere flüssige Ölkomponenten, kosmetische Wirkstoffe, Duftstoffe und Emulgatoren.

Geeignete weitere flüssige Ölkomponenten sind bereits vorher genannt worden. Kosmetische Wirkstoffe können z.B. Lichtschutzmittel (UV-Filtersubstanzen), Pflanzenextrakte, Vitamine, entzündungshemmende Wirkstoffe, Sebostatika oder Hautfeuchtigkeitsregulatoren sein. Badeöle enthalten zur Erhöhung der Spreitwirkung öllösliche Emulgatoren, z.B. Anlagerungsprodukte von 2 - 4 Mol Ethylenoxid an lineare Fettalkohole mit 12 - 16 C-Atomen. Auch Abschmink- und Reinigungsöle können solche Emulgatoren enthalten, um eine Abwaschbarkeit der Öle von der Haut durch Wasser zu erreichen.

Die folgenden Beispiele sollen den Gegenstand der Erfindung näher erläutern ohne ihn hierauf zu beschränken.

...

- 8 -

B e i s p i e l e

1. Herstellung von n-Decylerucat (techn.)

Eine Mischung aus 320 g (2 Mol) n-Decanol-1 (Lorol[(R)] C10) und 671,5 g (2 Mol) Erucasäure (Edenor[(R)] U 122) wurde nach Zusatz von 3 g feinem Zinnpulver als Katalysator in einem 2 l-Dreihalskolben mit Rührer, Thermometer, Stickstoffeinleitrohr und Destillationsaufsatz so lange auf 200°C - 220°C erhitzt, bis ca. 36 ml Wasser sich abgeschieden hatten. Nach ca. 6 Stunden war die Veresterung beendet. Der rohe Ester wurde mit aktivierter Tonerde versetzt, von der Tonerde und dem Katalysator abfiltriert und durch kurzes Andestillieren (200°C; 0,5 mbar) von flüchtigen Bestandteilen befreit.

Es wurde ein gelbliches, klares, geruchloses Öl mit den folgenden technischen Daten erhalten:

| | | |
|---|---|---|
| Säurezahl | : | 0,3 |
| Verseifungszahl | : | 117,2 |
| Jodzahl | : | 52,2 |
| Pourpoint | : | + 4 °C (DIN iso 3016) |
| Cloudpoint | : | + 16 °C (DIN iso 3015) |
| Flammpunkt | : | + 266 °C (DIN iso 2592) |
| Viskosität (20°C) | : | 25,3 mPa.s (Höppler-Kugelfall-Viskosimeter) |
| $D_{20}$ | : | 0,862 |

Die eingesetzten Rohstoffe hatten folgende Kenndaten: (In Klammern sind die Grenzen der technischen Spezifikation angegeben).

...

Lorol<sup>(R)</sup> C10 :   Hydroxylzahl : 350,6      (345 - 356)

Zusammensetzung (Gew.-%)

n-Octanol-1 :     1 %      ( 0 - 2 % )

n-Decanol-1 :    98 %      (94 - 98% )

n-Dodecanol-1 :   1 %      ( 0 - 4 % )

Edenor<sup>(R)</sup> U 122: Säurezahl : 167      (165 - 175)

Jodzahl    :   76      ( 70 -   76)

Zusammensetzung (Gew.-%)

| Erucasäure | : | | 92 % |
|---|---|---|---|
| Gadoleinsäure | : | ca. | 5 % |
| Ölsäure | : | ca. | 2 % |
| Stearinsäure | : | ca. | 0,5 % |
| Palmitinsäure | : | ca. | 0,5 % |

2. Anwendungsbeispiele

2.1 Hautcreme (Nährcreme), Typ O/W

| | | |
|---|---|---|
| n-Decylerucat (techn.) nach Beispiel 1 : | 20 | Gew.% |
| Palmitin/Stearin-Mono-/Diglycerid (Cutina<sup>(R)</sup>MD) : | 18 | Gew.% |
| Cetyl-/Stearylalkohol + 12EO (Eumulgin<sup>(R)</sup>B1) : | 1,5 | Gew.% |
| Cetyl-/Stearylalkohol + 20EO (Eumulgin<sup>(R)</sup>B2) : | 1,5 | Gew.% |
| Glycerin : | 5,0 | Gew.% |
| Cremogen<sup>(R)</sup> Hamamelis : | 5,0 | Gew.% |
| Triethanolamin : | 1,0 | Gew.% |
| Wasser : | 47,7 | Gew.% |
| Nipasteril<sup>(R)</sup> 30K : | 0,3 | Gew.% |

### 2.2 Hautemulsion (Vitamin-Hautemulsion) Typ O/W

| | |
|---|---|
| n-Decylerucat (techn.)( nach Beispiel 1) : | 12,0 Gew.% |
| Palmitin-/Stearinsäure-Mono-/Diglycerid (Cutina$^{(R)}$ MD) : | 5,0 Gew.% |
| Cetylalkohol (Lanette$^{(R)}$ 16) : | 2,0 Gew.% |
| Cetyl-/Stearylalkohol + 12 EO (Eumulgin$^{(R)}$ B1) : | 1,5 Gew.% |
| Cetyl-/Stearylalkohol + 20 EO (Eumulgin$^{(R)}$ B2) : | 1,5 Gew.% |
| Paraffinöl, dickflüssig : | 4,0 Gew.% |
| Vitaplant$^{(R)}$ öllöslich : | 2,0 Gew.% |
| Glycerin : | 5,0 Gew.% |
| Wasser : | 64,7 Gew.% |
| Vitaplant $^{(R)}$, wasserlöslich : | 2,0 Gew.% |
| Nipasteril 30 K : | 0,3 Gew.% |

### 2.3 Nährcreme (Typ W/O)

| | |
|---|---|
| n-Decylerucat (nach Beispiel 1) : | 20,0 Gew.% |
| Dehymuls$^{(R)}$ E (Pentaerythrit-Fett-säure-Zitronensäure-Fettalkohol-Mischester) : | 8,0 Gew.% |
| n-Decyloleat (Cetiol$^{(R)}$ V) : | 5,0 Gew.% |
| Bienenwachs : | 2,0 Gew.% |
| Vaseline (weiß) : | 10,0 Gew.% |
| Glycerin : | 3,0 Gew.% |
| Wasser : | 51,8 Gew.% |
| Nipasteril 30 K : | 0,2 Gew.% |

### 2.4 Sonnengel (Mikroemulsion, transparent)

| | |
|---|---|
| n-Decylerucat (nach Beispiel 1) : | 3,0 Gew.% |
| Paraffinöl, dickflüssig : | 3,0 Gew.% |
| Polyol-Fettsäureester (Cetiol$^{(R)}$ HE) : | 25,0 Gew.% |

...

Cetyl-/Stearylalkohol + 30 EO
(Eumulgin$^{(R)}$ B 3)                          :    12,0 Gew.%
Parsol$^{(R)}$ MCX                              :     2,0 Gew.%
Eusolex$^{(R)}$ 161                             :     2,0 Gew.%
Wasser                                          :    53,0 Gew.%

2.5  Hautöl (Sonnenöl)

n-Decylerucat (Beispiel 1)                      :    50,0 Gew.%
Capryl-/Caprinsäure-triglycerid
(Myritol$^{(R)}$ 318 )                          :    26,0 Gew.%
Karottenöl                                      :     2,0 Gew.%
Paraffinöl, dickflüssig                         :    20,0 Gew.%
Parsol MCX                                      :     2,0 Gew.%

2.6  Lippen-Pflegestift

n-Decylerucat (Beispiel 1)                      :    11,0 Gew.%
Cutina$^{(R)}$ LM (Mischung aus Fettalkoholen, Wachsen und Ölen)                        :    84,0 Gew.%
Karottenöl                                      :     5,0 Gew.%

Die in den Beispielen verwendeten Marken-Produkte sind von folgenden Herstellern erhältlich:

Lorol$^{(R)}$ C10              von Fa. Henkel KGaA
Edenor$^{(R)}$ U 122          von Fa. Henkel KGaA
Cutina$^{(R)}$ MD             von Fa. Henkel KGaA
Eumulgin$^{(R)}$ B1           von Fa. Henkel KGaA
Eumulgin$^{(R)}$ B2           von Fa. Henkel KGaA
Eumulgin$^{(R)}$ B3           von Fa. Henkel KGaA
Dehymuls$^{(R)}$ E            von Fa. Henkel KGaA
Cetiol$^{(R)}$ V              von Fa. Henkel KGaA

...

| Cetiol$^{(R)}$ HE | von Fa. Henkel KGaA |
| Myritol$^{(R)}$ 318 | von Fa. Henkel KGaA |
| Cremogen$^{(R)}$ Hamamelis | von Fa. Haarmann & Reimer, Holzminden |
| Nipasteril$^{(R)}$ 30 K | von Fa. Nipa Laboratories |
| Vitaplant$^{(R)}$ | von Fa. Chem Lab. Kurt Richter, Berlin |
| Karottenöl | von Fa. Chem. Lab. Kurt Richter, Berlin |
| Parsol$^{(R)}$ MCX | von Fa. Givaudan S.A., Genf (CH) |
| Eusolex$^{(R)}$ 161 | von Fa. Merck, Darmstadt |

Patentansprüche

1. Ölkomponente für kosmetische und/oder pharmazeutische Zubereitungen, dadurch gekennzeichnet, daß sie ganz oder überwiegend aus n-Decylerucat besteht.

2. Ölkomponente nach Anspruch 1, dadurch gekennzeichnet, daß sie wenigstens 80 Gew.-% n-Decylerucat neben kleineren Mengen von 1 - 20 Gew.-% an Estern von gesättigten und ungesättigten Fettsäuren mit 16 - 20 C-Atomen mit gesättigten Fettalkoholen mit 6 - 12 C-Atomen enthält.

3. Kosmetische Cremes und Emulsionen mit einem Gehalt an 5 - 40 Gew.-% einer bei 20°C flüssigen Ölkomponente, dadurch gekennzeichnet, daß 50 - 100 Gew.-% dieser Ölkomponente aus n-Decylerucat besteht.

4. Wasserfreie, flüssige kosmetische und/oder pharmazeutische Zubereitungen, dadurch gekennzeichnet, daß diese

20 - 60 Gew.-% n-Decylerucat
20 - 60 Gew.-% Paraffinöl

sowie gegebenenfalls weitere flüssige Ölkomponenten, kosmetische Wirkstoffe, Duftstoffe und Emulgatoren enthalten.